# EUROPEAN PATENT SPECIFICATION

(11) **EP 2 563 448 B1**
(45) Date of publication and mention of the grant of the patent: **12.10.2016**
(21) Application number: 10850628.8
(22) Date of filing: 29.08.2010
(51) Int. Cl.: A61M 31/00, A61M 37/00

(54) **METHOD AND APPARATUS FOR EXTENDING A TUBE**
VERFAHREN UND VORRICHTUNG ZUR VERLÄNGERUNG EINES ROHRS
PROCÉDÉ ET APPAREIL D'EXTENSION D'UN TUBE

(30) Priority: 28.04.2010 US 328656 P
(43) Date of publication of application: 06.03.2013
(73) Proprietor: Torus Medical Ltd, Beer-Sheva 84862 (IL)
(72) Inventor: LEVIN, Boris, 76283 Rehovot (IL); LEVIN, Bnaiahu, 74045 Ness Ziona (IL); ULANOVSKY, Levy, 763270 Rehovot (IL); LERNER, Vlad, 76283 Rehovot (IL)
(74) Representative: Ter Meer Steinmeister & Partner
(86) International application number: PCT/IL2010/000706
(87) International publication number: WO 2011/135557

(56) References cited:
- WO-A1-2010/020985
- WO-A2-01/70297
- US-A- 4 043 345
- US-A- 4 493 711
- US-A- 5 312 343

## Description

The present invention relates generally to an apparatus for extending a tube, particularly suitable for colon cleansing devices and methods, and more specifically to colon cleansing by supplying washing liquid into the colon and collecting drain waste flowing out.

### BACKGROUND OF THE INVENTION

There are many situations in which it is desired to insert a tube or pipe, particularly a flexible tube, into a relatively long and narrow channel or lumen or outer pipe. Such tube can be used to deliver fluids or an instrument to the end of the channel for medical and mechanical uses. At present, methods for such delivery include using guide wires or using tubes which are semi-rigid for ease of insertion. Common medical uses of such tubes include various endoscopic procedures, catheterization, and cleansing of the colon before performing colonoscopy examinations.

One of the most unpleasant and difficult stages of colonoscopy examination is the preparation of the patient prior to the examination. This preparation involves cleansing the patient's bowels and colon. During a colonoscopy procedure itself, patients are sedated so that they do not feel any pain, and sometimes do not even remember the test. However, the preparation is usually performed at home, and it can be quite challenging.

When using ingested laxatives, it can require the consumption of large volumes and/or of distasteful products. The exact laxative menu which is used varies according to the physician's or patient's experience, taste, and preference. One consequence of this often unpleasant, and occasionally unsuccessful, preparation experience is that there are patients who undergo colonoscopy, the preparations for which are imperfect or suboptimal. Poor preparation impairs the detection of colonic neoplasms, particularly small lesions, but even large tumors can be missed due to solids in the colon under examination.

The main advantage of laxatives, used in the conventional method for colon cleansing, is that they cause cleansing in the normal direction of elimination of solids and liquids in the colon, that is, from the small intestine toward the anus. In this direction, the fecal matter is eliminated from the body, in a way similar to that in normal human bowel movement activity.

Mechanical cleansing methods, such as an enema and hydrotherapy irrigation, introduce fluids from the rectum in the direction of the small intestine, tat is, in the direction opposite to the normal flow. These fluids are limited in their ability to reach far up the colon, and typically cleanse only about the lower third of the colon.

A number of prior art devices are known in the art which describe colonic cleansing in order to dislodge and remove fecal material from the patient's colon. These include:
U.S. Patent No. 4,182,332, which shows an insertable rectal catheter with a series of flanges contacting the rectal mucosal tissue. Such flanges would be likely to cause leakage, irritation, and infection of the tissue.

U.S. Patent No. 4,067,335 provides a fecal matter collecting unit with an insertable funnel with a series of ribs contacting the rectal mucosal tissue. These ribs allow leakage and put too much pressure on the tissue adjacent to each rib, which could cause irritation and infection. U.S. Patent No. 5,741,239 provides a fecal collection receptacle and tapered neck, a broad-lipped sealing ring with a bottom broad smooth flat sealing rim surface for contacting the rectal mucosal tissue that needs an external device for insertion and takes much space, causing uncomfortable feeling in the rectal area.

U.S. Patent No. 5,941,860 provides a fecal collector which comprises an elongated, flaccid pouch having an entrance end; an anchor attached to the pouch entrance end to anchor the entrance end in the lower bowel; and a positioner attached to the pouch in spaced relation to the anchor O-ring, to remain outside the lower bowel and adjacent to the body, for blocking tilting of the anchor in the bowel. This arrangement needs an external device for insertion and takes much space, which causes an uncomfortable feeling in the rectal area.

Lubricants or gels have been used to aid the insertion of devices through the rectum or anal canal area. U.S. Patent No. 3,881,485 to Davis, Jr. ("Davis") discloses a device for insertion through the anus into the upper rectum for the purpose of wiping the walls of the rectum clean of feces and stopping and retaining feces in the colon and rectum at a distance from the anus. The invention is a preformed fiber device that is shaped for insertion through the anus into the upper rectum. The device is inserted through the anus and up through the rectum with a coating of non-irritating lubricant applied to the wiper. The lubricant should preferably be an organic, inert, water soluble gel, but other suitable lubricants may be used.

Other prior art patents disclose colon cleaning systems and methods that introduce an enema solution (i.e., preferably with a laxative) into the colon through the anal opening via a suitable tube held in the rectum by an inflated bladder or balloon. The balloon and a tube are introduced into the body of the patient (i.e., via the anus and to the rectum/colon). U.S. Patents Nos. 4,403,982 to Clayton, 4,406,655 to Clayton, and 4,842,583 to Majlessi provide examples of such devices. However, these bladder or balloon devices require regulation of the pressure to the bladder or balloon, and they have the problem of the possibility of injury occurring to the patient if the pressure is not regulated properly.

U.S. Patent No. 5,049,138 to Chevalier et al. ("Chevalier") discloses a catheter having a tip that dissolves inside the body. The catheter includes a flexible tubular member that has an inner lumen and a rigid solid tip disposed at the end of the inner lumen. The tip (i.e. cone shaped) is formed of a material that is slippery when wet, soluble in bodily fluids and capable of absorbing radiographic fluids that are injected into the inner lumen for identification of the location of X-rays. A narrow passageway is disposed in the tip and is adapted to receive a guide wire for insertion of the catheter into an internal organ.

Other devices include those disclosed in U.S. Patent 6984226 of Abell et al, U.S. Patent 5,190,519 of Mead et al, U.S. Patent 5,176,630 of Shilling, et al., U.S. Patent 5,405,319 of Abell et al., U.S. Patent 5,019,056 of Lee et al., and U.S. Patent 4,874,363 of Abell. The primary purpose of each of these devices is the delivery of liquid into the colon through the anus of a patient for dislodging fecal material that may be lodged therein and then removing or draining the dislodged material along with the waste liquid from the colon to evacuate the bowels of the patient. Patent Application No. US 2007/0015965 of Cox et al, states that the cleansing of the colon for colonoscopy purposes needs to enter physically deep into the colon, however the method described uses a semi rigid tube, which is the same as performing an actual colonoscopy in parallel or before the regular colonoscopy.

There is known from US patent 6,988,988 an apparatus for endoscopic inspection including an anchor unit for anchoring outside a body tract, a flexible sleeve coupled to the anchor and having a distal end fixed to an internal unit adapted to be propelled through the gastrointestinal tract. The sleeve is held initially in a compact state (accordion folds) in the internal unit and is arranged to feed out from the internal unit as the internal unit is advanced into the gastrointestinal tract.

There is still a need for patient-friendly yet efficient colon cleansing devices and methods that overcome the limitations of the prior art devices and methods. In particular, there is a need for an easy yet efficient method for washing a colon in the direction from the cecum towards the anus.

Delivery of a contrast agent, such as barium sulfate, into the colon is well known. Also known is combined delivery of gas and contrast agent, see US patent 5,322,070. However, control over which specific portion of the colon is filled with barium remains limited. Furthermore, at present, in case of combined delivery of both barium and gas into the colon, one problem is how to control the properties of the barium coating of the colon wall, when the colon is filled with gas.

Conventional endoscopy uses cameras for imaging, often by way of taking two-dimensional pictures. Three-dimensional imaging in endoscopy is also known. See, for example, US patents 6,503,195, 6,798,570, 6,949,069, 6,749,346, 6,563,105, 5,751,341, 5,673,147. A need exists for delivery of a camera into a lumen of a patient using a simple and self-guided technique. Also, after delivery, a need exists for providing images of the lumen in a simple and informative way.

WO 01/70297 discloses an apparatus incorporating an elongate hollow element for being positioned along a body cavity of a patient. The hollow element has a leading region and trailing region and is arranged for being progressively everted along the hollow element from the leading region to thereby being increasingly extended for progressively lining the cavity as the trailing region follows along.

### SUMMARY OF THE INVENTION

The present invention relates to an apparatus for extending a tube as further disclosed in claim 1, the apparatus including a foldable tube having a folded portion inside an unfolded portion of the tube, and a source of fluid coupled to the unfolded portion for providing fluid into the unfolded portion to unfold and extend the folded portion out of the unfolded portion.

The invention further relates to an apparatus which enables cleansing of the colon in the normal direction, that is, from the cecum to the anus, yet using an apparatus which is introduced via the anus. In particular, there is provided an apparatus for cleansing a colon of a patient, the apparatus including a feed tube having a folded portion inside an unfolded portion of the tube, a fluid source arranged to feed fluid into the feed tube, thereby advancing the feed tube into the colon of the patient by inflation of the feed tube with fluid by unfolding, and at least one aperture in the folded portion permitting outflow of fluid from the feed tube.

Preferably, inflation of the feed tube causes an unfolded part the feed tube to increase in length with little change in width.

According to some embodiments of the invention, the increase in the length of the feed tube occurs due to sequential unfolding of a folded part of the feed tube to form an unfolded part of the feed tube. According to preferred embodiments of the invention, the increase in the length of the feed tube is performed by eversion (turning inside-out) of a folded part of the feed tube to form an unfolded part of the feed tube.

There is also provided, a method of extending a tube, the method including folding a tube so that a folded portion of the tube is located inside an unfolded portion of the tube, coupling a source of fluid to the unfolded portion, and providing fluid into the unfolded portion to unfold and extend the folded portion out of the unfolded portion.
There is further provided, a method of washing a colon of a patient having an anus, the method including inserting a flexible, foldable and inflatable feed tube into the colon of the patient through the anus, pumping a washing liquid into the colon through the feed tube, advancing the feed tube into the colon of the patient by inflation of the feed tube with the washing fluid, and draining the washing liquid from the colon via the anus.

It is an object of the present invention to provide a device for advancing a rube using fluid pressure, particularly suitable for use in colon cleansing devices.

It is an object and advantage of the present invention to provide a long soft sleeve or feed tube that unfolds, advancing into the colon, and conducts cleansing liquid through this feed tube deep into the colon. This fluid, then flows outside of the feed tube, to wash the colon with the cleansing liquid in the direction from the cecum toward the anus of the patient, thus providing a stream of liquid for the purpose of cleansing, one way in the direction from the cecum towards the rectum.

It is a further aspect of the present invention to provide a device for drainage of the washing liquid out of the body of the patient.

It is a further aspect to provide a method of fording the long soft feed tube prior to the procedure for ensuring an efficient way of unfolding the tube while moving inside the colon.

It is an object of the present invention to overcome the problems and limitations of the prior art that have been discussed. It is also an object of the present invention to be able to apply the principles and advantages of this invention to other related applications (i.e. including but not limited to animals).

According to embodiments of the present invention, external objects, such as a camera, can be inserted into the colon following the insertion of the feed tube into the colon or carried on the folded end of the feed tube.

According to other embodiments of the present invention, a capsule is connected to the feed tube, enabling it to reach the inside of the colon up to the cecum, and then disconnected to enable the capsule natural movement toward the anus or to pull it out using the feed tube.

An object of some embodiments of the present invention is to deliver a camera into a lumen of a patient using an inflating feed-tube. Another object is to capture images of the lumen during the subsequent withdrawal of the feed-tube from the lumen.

One object of additional embodiments of the invention is to improve control over which specific portion of the colon or other lumen is filled with a contrast agent, e.g. barium. Another object of these embodiments is to improve control over coating properties by the contrast agent when the colon is filled with gas.

According to further embodiments, the unfolded tube can massage a wall of the lumen.

### BRIEF DESCRIPTION OF THE DRAWINGS

The invention will now be described in connection with certain preferred embodiments, illustrating colon cleansing, for which it is particularly suited, by way of non-limiting example, with reference to the following illustrative figures so that it may be more fully understood.

With specific reference now to the Figures in detail, it is stressed that the particulars shown are by way of example and for purposes of illustrative discussion of the preferred embodiments of the present invention only and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
Fig. 1 is a schematic illustration of a tube partially inserted through a lumen, constructed and operative in accordance with the present invention;
Figs. 2A, 2B and 2C are schematic cross-sectional illustrations of a folded feed tube inside its container, in accordance with different embodiments of the present invention;
Fig. 2D is a schematic side view illustration of a feed tube inserted into a drainage channel with the help of a straw-like tube, in accordance with some embodiments of the present invention;
Fig. 3A is a schematic cross-sectional illustration of the unfolding feed tube, which has been packed in a concentric zigzag manner, in accordance with an embodiment of the present invention;
Fig. 3B is a schematic cross-sectional illustration of an unfolding feed tube, which was packed in an up and down zigzag manner, in accordance with an embodiment of the present invention;
Fig. 4 is a schematic side view illustration of a fully unfolded feed tube, in accordance with an embodiment of the present invention;
Figs. 5A-5E are schematic illustrations of five stages of insertion of the apparatus into the colon, in accordance with an embodiment of the present invention; Figs. 5A to 5E schematically illustrate several embodiments of the proposed methods of colon cleansing and the devices involved.
Fig. 6 is a schematic illustration of a device according to the present invention, showing inflow of feed fluid and the outflow of drainage;
Figs. 7A-7C are schematic illustrations of a side view of the system with the following variations:

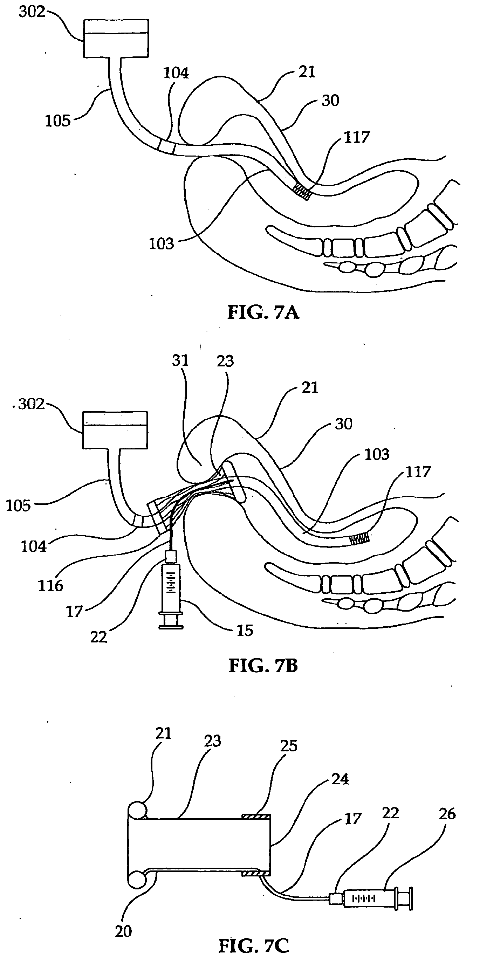

   Fig. 7A shows a feed tube alone, without any drainage channel in accordance with an embodiment of the present invention;
   Fig. 7B shows a feed tube with a soft drainage channel that carries an inflated balloon for keeping the channel in the anus in accordance with an embodiment of the present invention;
   Fig. 7C is a schematic illustration of a cross sectional view of an enema inflated when outside a patient, in accordance with an embodiment of the present invention;

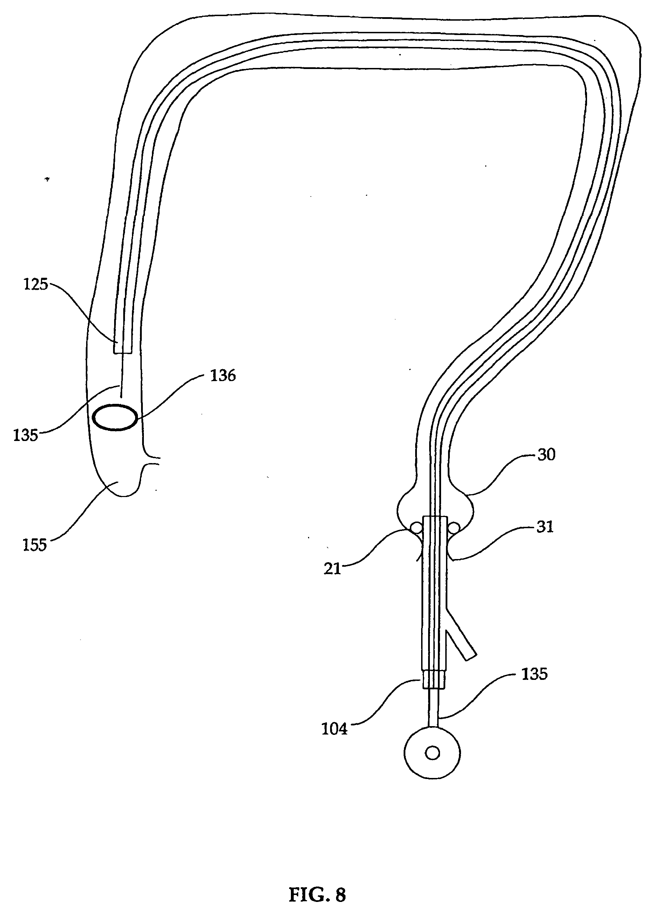

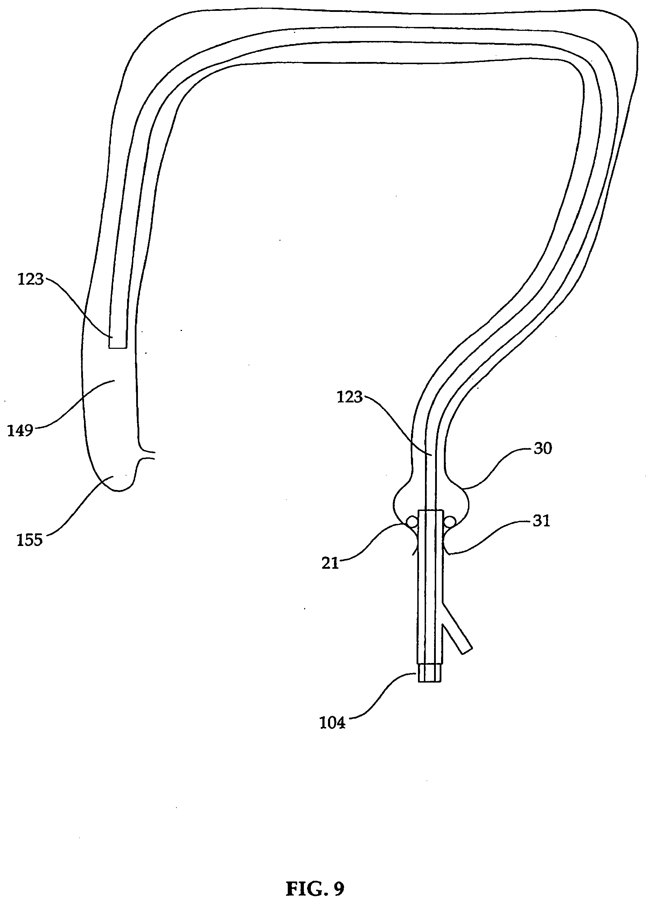

Fig. 8 is a schematic illustration of an external object, such as a camera or a detachable capsule, inserted through a tube in a lumen, in accordance with an embodiment of the present invention; Fig. 9 is a schematic illustration of an object, such as a camera or a detachable capsule, attached to a feed tube extended through a lumen, in accordance with an embodiment of the present invention;

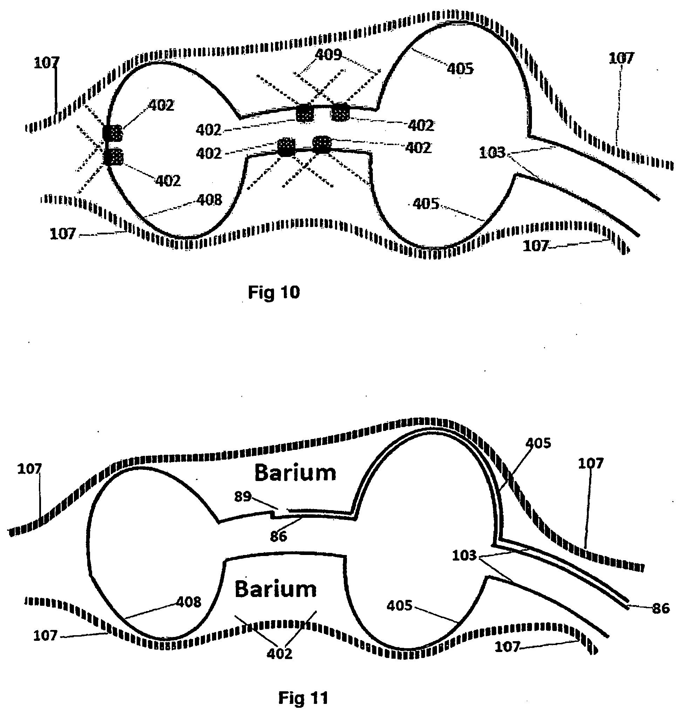

Fig. 10 is a schematic illustration of a lumen into which a 3D camera is introduced according to the present invention; and
Fig. 11 is a schematic illustration of a lumen into which a contrast agent is introduced, according to embodiments of the invention. In all the figures similar reference numerals identify similar parts.

### DETAILED DESCRIPTION OF PREFERRED EMBODIMENTS

In the detailed description, numerous specific details are set forth in order to provide a thorough understanding of the invention. However, it will be understood by those skilled in the art that these are specific embodiments and that the present invention may be practiced also in different ways that embody the characterizing features of the invention as described and claimed herein.

The present invention relates to an apparatus and method for extending a tube through a lumen or other pipe under pressure from a source of fluid flowing through the tube. The tube may carry only fluid, for example, cleansing fluid or medications, or may carry a device to be delivered to a location inside the lumen or pipe, such as a camera, or any other substances or objects, such as pills, powder, radiation sources, etc. This accomplished by providing a foldable tube having a folded portion inside an unfolded portion of the tube, where the fluid flowing into the unfolded portion causes the unfolded portion to unfold and extend out of the unfolded portion.

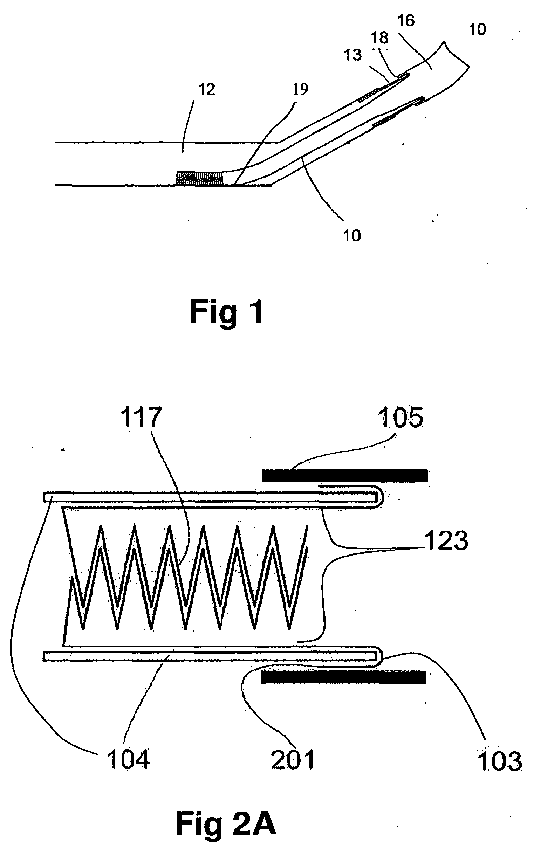

Referring now to Fig. 1, there is shown a schematic illustration of a tube or feed tube 10, constructed and operative in accordance with the present invention, partially inserted through a lumen 12. Tube 10 is originally folded inside itself and inside a container 13. Container 13 is coupled to a source of fluid 16. When it is desired to extend tube 10, for example, through a pipe or lumen 12, fluid from fluid source 16 is permitted to enter the unfolded end 18 of tube 10. Preferably, unfolded end 18 is wrapped around the edge of container 13, so as to anchor the tube and prevent it from sliding off container 13 when fluid flows therethrough. As described in detail hereinbelow, tube 10 is folded inside itself in such a way that, as fluid gradually flows therethrough, folded portion 19 adjacent the unfolded portion 18' gradually unfolds outwards from the folded portion of the tube, and becomes an extension of the already unfolded portion, thus extending the unfolded portion lengthwise. Preferably, tube 10 is formed of material of sufficient strength and flexibility that it extends as tube 10 unfolds, without substantially changing its diameter.

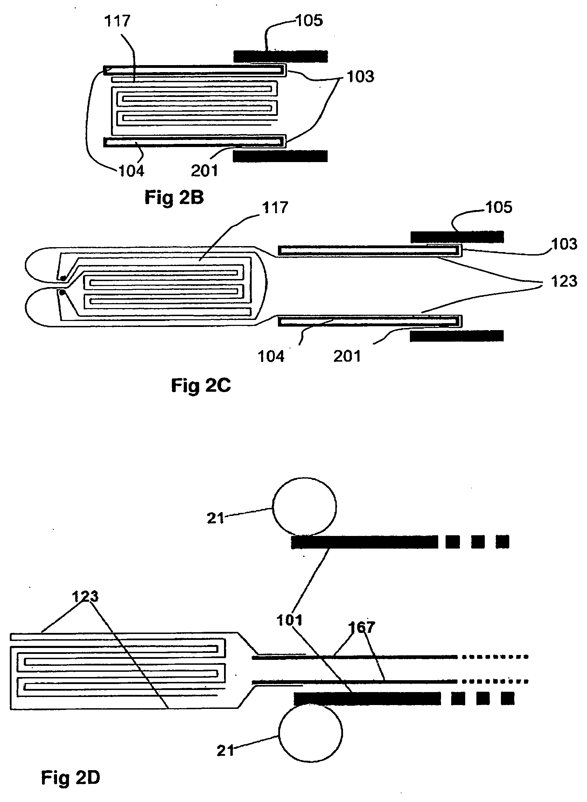

Figs. 2A, 2B and 2C schematically show three examples of possible packing geometries of a feed tube 103 pre-packed inside a cylindrical container 104 before feed tube 103 is inflated. In each of the three figures, the proximal end portion 201 of feed tube 103 is folded over the proximal (right) edge of container 104. Thus, feed tube 103 starts at its end portion 201, folded over the right edge of container 104, and is aligned along the inner walls of container 104 towards the left end of container 104. This portion is already unfolded and is indicated as unfolded part 123 of feed tube 103. Feed tube 103 further continues as folded (packed) part 117 of feed tube 103. As can be seen in Figs. 2A, 2B and 2C, folded (packed) part 117 of feed tube 103 is disposed inside unfolded part 123 of feed tube 103.

There are alternatives to using a container 104 for the introduction of feed tube 103 into a lumen. One alternative is a semi-rigid tube that looks and feels very much like a straw commonly used for drinking from a cup. As shown in Fig. 2D, straw-like tube 167 carries feed tube 103 at its distal (left) end, so that feed tube 103 can be inflated through straw-like tube 167. Where is it desired to drain the fluid, during extension or once the feed tube has been fully extended, the feed tube can be inserted into the lumen through a drainage channel 101 disposed in the lumen. Straw-like tube 167 is introduced into drainage channel 101 and is pushed further through channel 101 until straw-like tube 167 sticks out of the distal (left) end of channel 101, as is seen in Fig. 2D. Preferably, straw-like tube 167 is much thinner than unfolded part 123 of feed tube 103. This difference in width leaves more room for drainage through drainage channel 101 compared to the situation in which unfolded part 123 of feed tube 103 is disposed inside drainage channel 101. Broken lines indicate that these lines continue beyond the frame of the drawing.

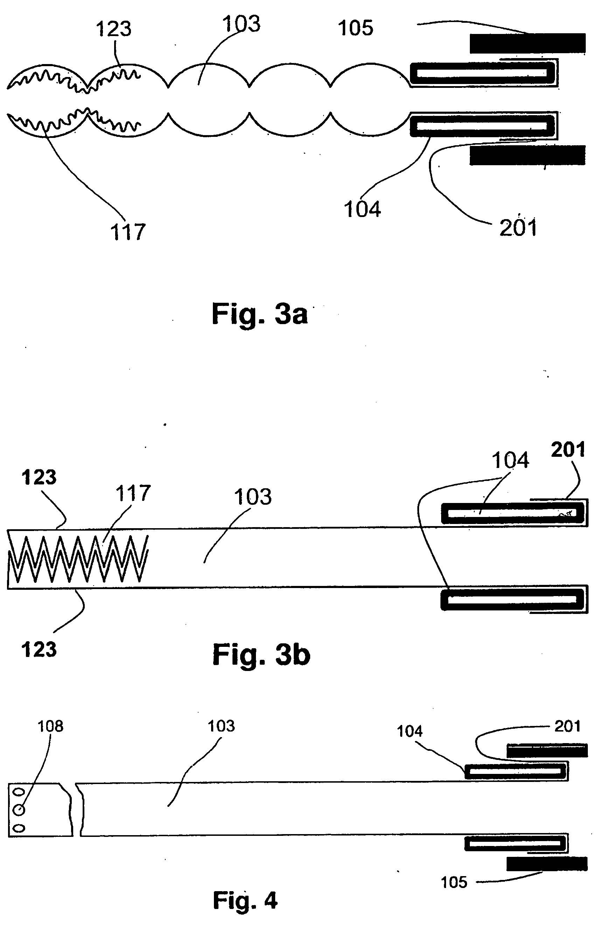

Fig. 3B schematically shows a zigzag structure of packed (folded) part 117 of feed tube 103 unfolding during the process of inflation of feed tube 103 by fluid from the fluid source (not shown), as feed tube 103 extends (advances) further and further to the left. The leftmost fold of folded part 117, inside unfolded part 123, everts (inverts) over the leftmost edge of unfolded part 123 of feed tube 103, turning inside-out. As it everts and unfolds, it gets inflated and becomes an additional segment of unfolded part 123 of feed tube 103. The leftward extension of unfolded part 123 of feed tube 103 pulls folded part 117 to the left accordingly. This is because the two parts, folded 117 and unfolded 123, represent topologically a single soft topologically cylindrical sleeve forming the feed tube 103. In other words, feed tube 103 comprises both its folded segment 117 and its unfolded segment 123. In further extension of feed tube 103 leftwards, this unfolding step (of a single fold) is essentially repeated again and again, sequentially, as feed tube 103 keeps turning inside out, like a sock.

While cylindrical in its topology, feed tube 103 does not necessarily have to be exactly cylindrical in shape. Fig. 3A shows a non-cylindrical shape option for feed tube 103. In Fig. 3A, at least a part of unfolded segment 123 of feed tube 103 has a cross-section that is quasi-periodically variable along the length of the sleeve. More specifically, that part of feed tube 103 comprises a plurality of bulging (widened) sections separated by, and thus alternating with narrowed necks, in a sausage-chain (bead-string) manner. Both the period and the width of the sections and necks may vary along the sleeve. Fig. 3A also illustrates folded part 117 of feed tube 103. Folded part 117 is illustrated as a concentric zigzag structure of variable width inside unfolded segment 123 of feed tube 103.

As an advantage, compared to cylindrical feed tube 103 of Fig. 3B, the sausage-chain (bead-string) structure of feed tube 103, shown in Fig. 3A, improves the flexibility and mobility of feed tube 103 during its extension through the lumen as tube 103 is being inflated. Thus, the sausage-chain shape eases the extension of feed tube 103 into a lumen that is not straight, for example, a curling colon. In addition, compared to the cylindrical shape of feed tube 103, a bead-string (sausage-chain) shape of feed tube 103 reduces the chances of tissue irritation caused by sharp corners at the kinks formed when cylindrical feed tube 103 bends.

As an illustration of both structure and process, Fig. 3A schematically shows also a part of the sausage-chain shaped structure that is not yet unfolded. Fig. 3A thus illustrates the transition from folded to unfolded state. Packed (folded) part 117 of feed tube 103 is unfolding during the process of inflation of feed tube 103, as feed tube 103 extends further and further to the left. A single step of this inflation process occurs as follows. The leftmost fold of folded part 117, inside unfolded part 123, everts (inverts) over the leftmost edge of unfolded part 123 of feed tube 103, unfolds, and inflates (i.e., fills with fluid) to become the rounded leftmost bead of the chain of beads of unfolded part 123 of feed tube 103, shown in Fig. 3A. The leftward extension of unfolded part 123 of feed tube 103 pulls folded part 117 to the left accordingly, because the two parts, 117 and 123, represent topologically a single soft cylindrical sleeve, feed tube 103. In further extension, this unfolding step (of a single fold) is essentially repeated again and again sequentially, as feed tube 103 keeps turning inside out, like a sock, just as in Fig. 3B.

Preferably, the material of which feed tube 103 is made is not stretchable significantly at pressures applied during its inflation. This material typically may have a texture similar to that of common sandwich bags. In this way, the feed tube can be unfolded/inflated without increasing substantially in diameter. At the same time, the feed tube material may have limited stretchability, to help it adapt to the bends and folds in the lumen that it is expanding through. A typical diameter of feed tube 103 for use in colon cleansing is 12 mm. Its length is typically smaller than the length of the colon from the anus to cecum.

It will be appreciated that the feed tube can be open at its distal end, so that the fluid inflating it can exit from the tube at the end. Alternatively, the distal end of the feed tube can be sealed, as shown in Fig. 4, with one or more exit holes or apertures 108 opening in feed tube 103. Then the fluid can flow out of the exit hole of feed tube 103 into the lumen or drainage channel. Alternatively, or in addition, apertures 108 of selected sizes can be formed in certain portions or along the length of feed tube 103, permitting outflow of fluid at pre-selected distances along the lumen.

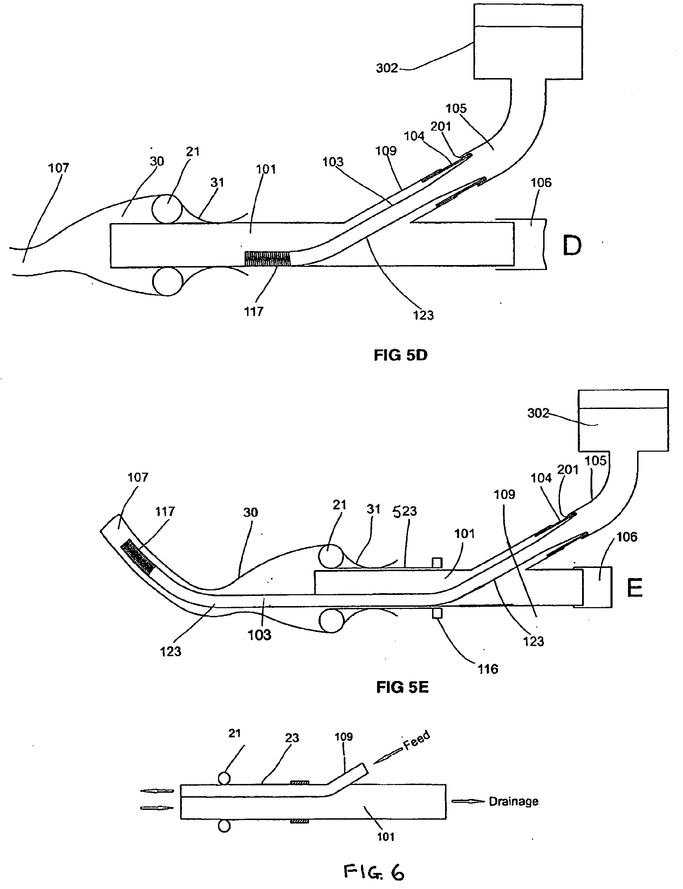

Different methods of insertion of the tube according to the invention into different lumen for different purposes will now be described, by way of non-limiting example only. For the purpose of cleansing (washing) the colon, and in other locations where drainage of inflation fluid is desired, a rigid drainage channel 101, also referred to as insertion tool 101, such as shown in Fig. 6, can be utilized. Rigid drainage channel 101 is essentially cylindrical, slightly narrowing towards its leading (distal) end, on the left, while widening towards its trailing (proximal) end, on the right. The feed tube 23 is inserted into the lumen via the drainage channel, which serves to support and guide the feed tube. All along its length, channel 101 is much wider than feed tube 23, which passes inside channel 101. In other words, the presence of feed tube 23 inside rigid drainage channel 101 leaves a lot of room for drainage, as shown schematically in Fig. 6.

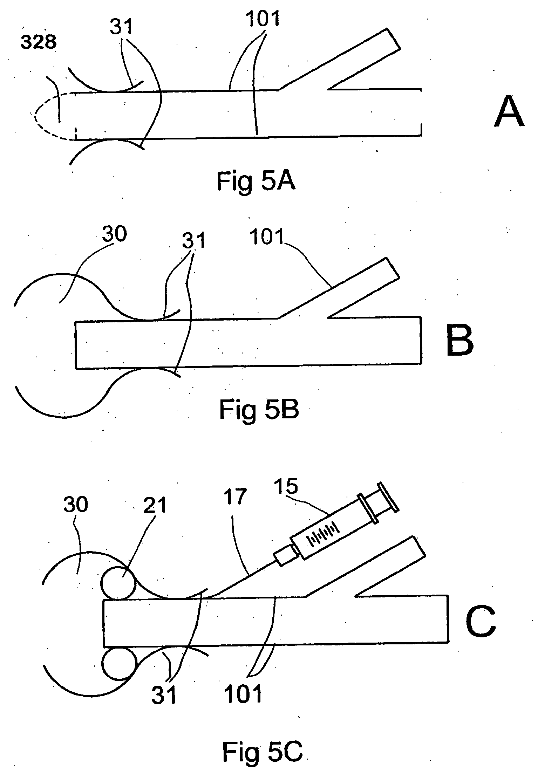

Figs. 5A to 5E schematically illustrate several embodiments of the proposed methods of colon cleansing and the devices involved. In these Figures, a rigid drainage channel 101, as described above, is employed to anchor the feed tube 103 in the rectum 31 of a patient. A working example of rigid drainage channel 101 has the following dimensions: the length is 14 cm; the outer diameter is 24 mm at the trailing (proximal) end, on the right, tapering down to 20 mm at the leading (distal) end, on the left; the wall thickness is 1.0 to 2.0 mm. Typically, the material of rigid drainage channel 101 is conventional rigid plastic.

A removable cap 328, seen in Fig. 5A, makes the insertion of rigid drainage channel 101 into anus 31 smooth because it covers the leading edge of rigid channel 101. One way to make cap 328 is by coating the leading edge of channel 101 with a formable material and letting the material harden. Alternatively, cap 328 is formed separately from channel 101. For example, cap 328 can be made of ice by filling a cap form (a mold) with water and letting the water freeze. Such separately made ice cap 328 is stored frozen and is inserted into the leading end of rigid drainage channel 101 just before usage. Cap 328 is made of a material that would melt, soften, or dissolve upon being inserted into anus 31. Preferably, the material of cap 328 is ice. The cap can cover the leading (left) edge of rigid channel 101 either on the inside or on the outside of channel 101, or both. When ice cap 328 covers the edge of rigid channel 101 on the inside, the ice fills channel 101 to the depth of a few millimeters from the leading (left) edge, as in Fig. 5A.

At the start of the procedure, rigid channel 101 is inserted into anus 31 with ice cap 328 sitting on its leading edge. Then warm, body temperature liquid is pumped via rigid channel 101 into the body, from right to left. The warm liquid pushes ice cap 328 out of rigid channel 101 left, into the body, and makes the ice cap melt, at least in part, as shown in Fig. 5B. Alternatively to ice, the cap material can be one of those used for common suppositories and its elimination upon insertion can have a different mechanism.

Another way to make the insertion of rigid drainage channel 101 smooth is to make cap 328 shown in Fig. 5A of a material more durable than ice, for example plastic. In this case, cap 328 should be removable from its place at the end of channel 101 and then be able to be withdrawn from the body of the patient. For this purpose, cap 328 may be composed of segments small enough to be pulled back through rigid drainage channel 101 with a flow of liquid. The cap falls apart as it is pushed out of rigid drainage channel 101 forward, to the left, by a wave of liquid generated in channel 101 for this purpose. The wave can be generated manually by pushing a piston, squeezing a bulb syringe, a fleet enema type device, or in a similar way. Then, as the liquid drains back to the right, out of rectum 30 through channel 101, the cap segments float (eventually, as more liquid drains out) with the liquid flow through rigid drainage channel 101 into the sewage.

In order to firmly anchor the drainage channel 101 in the anus 31 and prevent leakage of fluid, an inflatable balloon 21 may be inserted inside rectum 30. In Fig. 5C, the part of rigid drainage channel 101 that is inside the rectum is attached to an inflatable balloon 21, which balloon is inflated inside the rectum by means of an external pump, in order to keep the drainage channel 101 from exiting from the anus. Fig. 5C shows a pump device 15, such as a syringe, for inflating balloon 21. Pressure-providing tube 17 conducts the inflating liquid or gas from pump device 15 to balloon 21. The diameter of pressure-providing tube 17 is typically between 0.5 and 2.0 millimeters. Tube 17 may be a fixed connection, e.g., inflation and deflation tube. In this way, pump device 15 can inflate the balloon to create the necessary seal at the start of the procedure, and can be used to deflate the balloon 21 at the end of the procedure. Optionally, a one-way valve (not shown) is installed between pump device 15 and balloon 21 for preventing balloon 21 from deflating prematurely. Manual opening of such a one-way valve causes balloon 21 to deflate when needed. Inflated balloon 21 together with rigid drainage channel 101 is wider than rigid drainage channel 101 alone, typically by 20 - 40 mm. In other words, inflated balloon 21 adds, typically 20 - 40 mm, to the width of rigid drainage channel 101. This extra width added by balloon 21 helps to keep rigid drainage channel 101 from exiting from anus 31. Preferably, balloon 21 is a ring shaped toroid encircling rigid drainage channel 101, as shown in Figs. 5C and 5E, close to the topologically circular leading (left) edge of channel 101. When ring shaped balloon 21 is deflated, it looks like an inflatable cuff around essentially cylindrical channel 101. Therefore, when balloon 21 is ring shaped, it can be referred to as inflatable cuff 21. During the insertion of rigid drainage channel 101 into anal canal 31, deflated and folded inflatable cuff 21 is preferably kept under removable cap 328 shown in Fig. 5A.

Ring shaped balloon 21 may be attached to rigid drainage channel 101 by means of sheath 523, seen in Fig. 5E. Sheath 523 is optionally glued to channel 101 or otherwise affixed to it. Alternatively, sheath 523 may be unattached to channel 101, at least part of the time. Sheath 523 can be either rigid or pliable, depending on the embodiment. The outer (proximal, rightmost) edge of sheath 523 is attached to hard ring 116 that encircles drainage channel 101 for ease of manual handling. Optionally, sheath 523 is absent. In this case, both ring shaped balloon 21 and hard ring 116 are attached directly to rigid drainage channel 101.

Optionally, the part of drainage channel 101 that is outside of the rectum is attached to another inflated balloon (not shown) for helping to keep drainage channel 101 from moving too far into the rectum. Specifically, such a balloon can be ring-shaped and attached to the area of sheath 523 seen in Fig. 5E between ring 116 and anus 31.

Before the inflation of feed tube 103 starts, feed tube 103 is packed in rigid container 104 inserted into rigid feed holder 109 that is branching out of rigid drainage channel 101 at a sharp angle backwards, as shown in Figs. 5D and 5E. With its inflation, feed tube 103 unfolds and extends out of container 104 via rigid feed holder 109 and further via drainage channel 101 into rectum 30 and still further into colon 107, as shown in Fig. 5E. The inner diameter of rigid feed holder 109 is typically slightly larger, for example by 1.0 or 2.0 mm, than the outer diameter of unfolded segment 123 of feed tube 103. The material of container 104 is typically conventional rigid plastic. Rigid feed holder 109 is typically integrally formed with, or firmly affixed to drainage channel 101 and is made of the same material.

Liquid for washing the colon is stored in a reservoir 302, as seen in Fig. 5D. A small hose 105 conducts the washing liquid from reservoir 302 drown into feed tube 103. The washing liquid flows down inside small hose 105 under the pressure of its own weight. In other words, the liquid is being pulled by the force of gravity. Alternatively, a pump (not shown) can be provided, if desired, to pump the fluid from reservoir 302 into feed tube 103. Feed tube 103 is shown in Fig. 5D to be advancing through drainage tube 101 into rectum 30.

The mechanism of the advance of feed tube 103 into colon 107 is by inflation and unfolding of feed tube 103, as feed tube 103 is being filled with the washing liquid from reservoir 302. The inflation pressure may be comparable to pressures used in common enemas and, more generally, is between about 0-1 atm higher than atm pressure. The inflation of feed tube 103 extends (increases the length of) feed tube 103 with little change in its width. The increase in the length of feed tube 103 occurs due to the sequential unfolding of folded part 117 of feed tube 103 into unfolded part 123 of feed tube 103. Preferably, the sequential unfolding is performed by eversion, in other words by turning inside-out of folded part 117 of feed tube 103 into unfolded part 123 of feed tube 103. It is essential that folded part 117 of feed tube 103 is located inside unfolded part 123 of feed tube 103. In other words, eversion is like turning a sock inside out. But unlike in a sock, unfolded part 123 of feed tube 103, the outer part, is inflated and assumes essentially the shape of a tube under the pressure of inflation, behaving like a soft hose. It will be appreciated that, in this way, feed tube 103 extends through the colon substantially without friction between feed tube 103 and the lumen walls. It is also important to minimize the friction between folded part 117 and unfolded part 123. This friction arises primarily at the turns of a curling lumen. It rises dramatically with the length of the contact (along the tube) between folded part 117 and unfolded part 123. Therefore, it is preferable to minimize this length by making the packing of folded part 117 as compact along the tube as possible. In other words, good packing should minimize the trailing of folded part 117 inside unfolded part 123. For example, it can easily be seen that the packing structure shown in Fig. 2A allows much more trailing as compared to those shown in Fig. 2B and Fig. 2C. In the preferred embodiments of this invention, the packing is maintained compact during the inflation of the feed tube. Specifically, the criterion of good packing is the following: during inflation, the maximal size achieved by folded part 117 should be much smaller than the maximal length of unfolded part 123.

In Fig. 5E, feed tube 103 is shown to be advancing via anal canal 31 into colon 107. In the process of its advance, feed tube 103 is being filled with the washing liquid from reservoir 302 and reaches a length at which an exit hole or holes, such as hole 108 in Fig. 7, opens in feed tube 103. Then the washing liquid flows out of the exit hole of feed tube 103 into colon 107 and washes colon 107 by flowing outside of feed tube 103 back towards the drainage channel, in the direction from the cecum to the anus. The washing liquid is drained out of rectum 30 via rigid drainage channel 101 and further via drainage port 106 through a hose out into sewage system. The washing liquid continues to flow inwards through feed tube 103 in the direction from anus 31 deep into colon 107 and continues to flow back outside feed tube 103 through colon 107 towards the anus, thus washing colon 107. It will be appreciated that the washing liquid can be arranged to flow out through feed tube into the colon at substantially any location along the colon. However, the arrangement of the present invention is particularly suited to providing washing liquid substantially to the cecum, thereby providing non-traumatic cleansing of substantially the entire colon, which is not possible with conventional lavage devices, which drive water up into the colon from the anus, so not much water reaches the cecum and which is problematic for bacterial flora preservation.

It will be appreciated that the pumping of the liquid via rigid channel 101 into the body, from right to left can be done in various ways. One way is to supply the liquid via rigid feed holder 109. Another way is to split the passage of liquid via drainage port 106 into two passageways - one for draining out, the other for pumping in. One way valves, clamps, etc. can be used to close one or both passages. The liquid for pumping in can be supplied from a reservoir 302. If a vigorous wave is needed to be pumped in, the wave can be generated manually by pushing a piston, squeezing a bulb syringe, a fleet enema type device, or in a similar way.

It will be appreciated that a rigid drainage channel is not always required or desired. Fig. 7A illustrates direct insertion of a feed tube 103 via the anus into the rectum, without the benefit of a drainage channel at all. The container 104 of feed tube 103 is coupled via a small hose 105 to a liquid reservoir 302. The flow of liquid from reservoir 302, under the force of gravity, causes the unfolded portion 117 of feed tube 103 to gradually unfold, as described above.

Fig. 7B illustrates a pliable (soft) drainage channel 23, which can be used instead of the rigid drainage channel 101 shown in Fig. 5E. Channel 23 is essentially a soft sleeve and is also referred to as pass sleeve 23. Like rigid drainage channel 101, pass sleeve 23 is used for draining the washing liquid and feces out of the body. Yet, unlike in rigid drainage channel 101, the drainage via pass sleeve 23 is accomplished, at least in part, by natural motion of the muscles of the rectum and sphincter, and by the natural opening of the anal canal. Pass sleeve 23 is shown to be attached to inflated toroidal ring-shaped balloon 21 that rims the circular edge of the distal end of channel 23. Ring-shaped balloon 21, also referred to as inflatable cuff 21, is inflated after it is inserted inside rectum 30 and keeps the leading end of pass sleeve 23 inside rectum 30.

Reference is now made to Fig. 7C, which is a schematic cross sectional view of pass sleeve 23 with attached toroidal ring-shaped balloon 21 when inflated outside of a patient, for demonstration purposes. For the purpose of drain waste disposal, proximal end 24 of pass sleeve 23 can be connected to a container (not shown) or a sewage hose. For enema purposes, the proximal end 24 of pass sleeve 23 can be connected to a bag filled with a liquid. For both drain disposal and enema purposes, together, both connections can be made to the same proximal end 24 of pass sleeve 23. The two hoses can be closed by clamping them, usually done alternatively, one at a time. Either way, the connection of proximal end 24 of pass sleeve 23 to downstream drain vessels is implemented by means of a conventional plumbing connector 25.

Toroidal ring-shaped balloon 21, when fully inflated, preferably has an inner diameter of between 30 and 80 millimeters. Its outer diameter is larger, typically by 5 to 40 millimeters, than its inner diameter. As in Fig. 7C, the diameter of pass sleeve 23 is typically close to the inner diameter of toroidal balloon 21 and, thus, ranges typically between 30 and 80 millimeters. The diameter of pass sleeve 23 does not have to be uniform along the sleeve, but rather can be designed to vary, depending on various requirements. Therefore, the cylindrical shape of pass sleeve 23 in Fig. 7C is only a non-limiting example. The inflated ring of toroidal balloon 21 does not have to be exactly circular, and neither do any of its cross-sections. Toroidal ring-shaped balloon 21 can be inflated with liquid to less than its full volume. Such partial inflation leaves balloon 21 soft enough to adapt itself to the shape of the body canal, such as rectum, in which it is located. This way, irritation of the canal wall is minimized.

Fig. 7B illustrates feed tube 103 passing inside pass sleeve 23 into rectum 30. Later, feed tube 103 is further extended by inflation deep into the colon similarly to the way it is extended through rigid drainage channel 101, also into the colon. Then, feed tube 103 conducts the washing liquid into the colon. To prevent the sphincter from squeezing pass sleeve 23 and, thus, closing the flow of liquid through feed tube 103, which passes inside pass sleeve 23, a rigid tube that is wider than feed tube 103 is inserted into pass sleeve 23 inside the anus. Feed tube 103 is passed inside this rigid tube, which is inside pass sleeve 23.

The rigid tube can be made wide enough to serve both for passage of feed tube 103 into colon 107 and for drainage from the rectum out. In this dual role, this rigid tube is very much like rigid drainage channel 101. Therefore, this rigid tube is referred to herein using the same term and numeral - rigid drainage channel 101. In the same procedure, drainage can be carried out via both rigid drainage channel 101 and pass sleeve 23. Pass sleeve 23 is preferable for draining large fecal bodies that may clog rigid drainage channel 101. By contrast, the latter is preferable for liquid drainage, as well as for small and medium size fecal bodies. If a large fecal body clogs rigid drainage channel 101, then a wave of liquid in the reverse direction, towards the rectum, is used to unclog channel 101. If necessary, the unclogging procedure is repeated until the clogging material goes out through pass sleeve 23.

Before the cleansing begins, rigid drainage channel 101 together with inflatable cuff 21 and the leading portion of pass sleeve 23 are inserted into anus 31 with the help of an ice cap or other removable cap, similar to cap 328, which is described above for rigid drainage channel 101. Part of pass sleeve 23 is lined up along the outer surface of rigid drainage channel 101. Vaseline is spread on the outside of this part of pass sleeve 23 for smoother insertion into anus 31.

The materials of feed tube 103, pass sleeve 23 and toroidal balloon 21 have limited stretchability, particularly under inflation pressures and other forces applied during the procedure. These materials typically have a texture similar to the texture of common sandwich bags. Non-limiting examples of suitable materials include polyethylene (preferably low density polyethylene), polypropylene, and polyurethane. The materials should be as biocompatible as reasonable and have no or minimal toxic or harmful effects. The thickness of these sheet materials typically ranges between 10 and 150 micrometers and is preferably about 40 micrometers. The thickness and the nature of the sheet materials of feed tube 103, pass sleeve 23 and toroidal balloon 21 do not have to be necessarily uniform along the surface. Multiple layered sheets can be used, especially for making balloon 21, where leaks are least desirable.

Methods for producing feed tube 103, pass sleeve 23 and toroidal balloon 21 can vary. Prefabricated sheets of the material can be purchased wholesale. The sheets can be heated by applying a properly shaped hot wire to make pieces of appropriate shapes. Either then or later, while the appropriate edges of the pieces are heated, they are stitched together, as needed, to form seams. Other common methods of production are by extrusion or dip molding. Preferably, the parts are disposable.

As stated above, a tube according to the invention that is advanced into a lumen or pipe by inflation with a liquid or a gas, can be used for purposes other than cleansing the colon. According to one embodiment of the invention, as Fig. 8 illustrates, tube 123, after being unfolded in the appropriate lumen, can be used as a channel for insertion of an endoscope 135 through tube 123 for delivering an object 136 to a desired location inside a patient's body. Object 136 can be, for example, a camera, a detachable capsule, a source of radiation, such as: light, X-rays, positron emission, other radioactivity, etc., or any other object to be delivered thereto.

According to another embodiment, as Fig. 9 illustrates, an unfolded tube 123 can, itself, be used as an endoscope. In this case, the object 149 is attached directly to tube 123 itself, for insertion through the lumen as the tube 123 unfolds. Unfolded tube 123 is shown here to carry object 149 attached at the end of tube 123, although object 149 could, alternatively, be attached at some other point along the length of tube 123. Object 149 can be, for example, a camera, a detachable capsule, a source of radiation, or any other appropriate object. In Fig. 9, tube 123 is preferably closed at its distal end. By contrast, in Fig. 8, and for washing purposes, tube 123 is open, preferably at the very (distal-most) end, for delivering object 149 or the washing liquid, respectively.

One specific application of tube 123 shown in Fig. 9 is visual inspection of the colon using a camera located in object 149. During the inspection, tube 123 is preferably gradually withdrawn from the colon, as in conventional endoscopy. For the latter application, tube 123 may include a section at its very end that is wide enough to expand the colon walls outwards for inspection. In other words, this wider section of tube 123 spreads the wall out for visual examination of the wall portion sliding off the wide section, as tube 123 is gradually withdrawn from the colon. The image seen by the camera can be transmitted wirelessly from object 149 to an appropriate receiver. The inspection can be performed after a video or still recording of the images from the camera is made.

Fig. 10 schematically shows a plurality of cameras 402 attached to a shaped tube 403. The delivery of cameras 402 into colon of other lumen 407 is by inflation of tube 403, its unfolding and advancing into lumen 407, as described above. The properties and handling of tube 403 are the same as described above for feed-tube 103. After complete inflation and advance of tube 403 into lumen 407, towards the left, Fig. 10 shows the withdrawal of tube 403 through lumen 407, towards the right. Balloons 405 and 408 in Fig. 10 are essentially two widened portions of tube 403. Balloon 408 is at the trailing (leftmost) end of tube 403. Balloon 405 is close to balloon 408 along tube 403, at a distance comparable to the characteristic diameter of the balloons, typically several centimeters. The two balloons, 405 and 408, engage the walls of lumen 407 to keep the walls at an appropriate distance from cameras 402 for camera 402 to take pictures of the walls.

Six cameras 402 are shown in the illustrated embodiment, in three pairs. The field of view of the cameras are indicated by two diverging punctured lines 409. In each of the three pairs of cameras shown in this example, the two cameras in the pair have overlapping fields of view. The overlap of the two fields of view allows a reconstruction of a three-dimensional (3D) image of the wall of lumen 407 (binocular parallax method). Alternatively, reconstruction of 3D images can be carried out from data collected from a single camera, rather than two cameras. For example, the second camera can be replaced with a mirror chain, while the image data are collected alternatively from one point of the chain and another. Yet another option is to reconstruct 3D image from single camera shots taken at different points of the trajectory of its withdrawal along the colon (motion parallax). Still another option is structured light depth extraction, such as described in US patent 6,503,195.

The image data from one or more cameras can be sent to an outside computer either wirelessly, i.e., via a transmitter, or by wire, such as an electric cord or optical fiber (not shown). In the example of Fig. 10, each camera 402 carries a simple source of light, such as a LED, for illumination of the lumen wall. An alternative is a source of structured light for depth extraction. Such source is preferably located away from the camera recording the image for depth extraction, yet can be attached to the same surface, such as that of balloons 405 and/or 408. The image data, whether still or video, whether 3D or not, can be viewed in real time or recorded for subsequent viewing. Descriptions of methods of 3D image reconstruction can be found in US patents 6,503,195; 6,798,570; 6,949,069; 6,749,346; 6,563,105; 5,751,341; and 5,673,147.

If the image data are to be recorded for subsequent viewing, the data collection can be performed by a nurse or technician, rather than by a doctor. The doctor's attention can subsequently be focused more fully on viewing, rather than on both viewing images and handling an endoscope at the same time. 3D image viewing can be done using artificial shading, virtual rotation, binocular glasses, light polarization glasses, or any other convenient method of 3D display. Computerized pre-processing of the 3D images can select and flag spots that may need extra attention by the human viewer.

Referring now to Fig. 11, there is shown a schematic illustration of the use of a tube 403 for delivery of a contrast agent for medical imaging to colon 107. In this example, the contrast agent is barium sulfate, also called "barium". Barium is commonly used as a suspension of fine particles in an aqueous solution in the medical imaging technique called "barium enema", a procedure for colon imaging. Other contrast agents, such as water soluble contrast agents, can be used instead of barium. Unlike in barium enema, here in Fig. 11, barium fills only a small portion of colon 107, between balloons 405 and 408. Balloons 405 and 408 are essentially two widened portions of tube 403. Balloon 408 is shown to be at the trailing (leftmost) end of tube 403. Balloon 405 is close to balloon 408 along tube 403, typically at a distance of several centimeters. The delivery of balloons 405 and 408 into colon 107 is by inflation of tube 403, its unfolding and advancing into colon 107, as described above. Upon the inflation and advance of tube 403 into colon 107, towards the left, Fig. 11 shows the withdrawal of tube 403 through colon 107, towards the right.

In Fig. 11, barium is shown to fill the colon between balloons 405 and 408. Thus, the segment of the colon filled with barium is well defined and controlled by the location of balloons 405 and 408. As balloons 405 and 408 are being withdrawn from colon 107 (towards the right), the barium filling moves with the balloons. During this withdrawal, barium can be intentionally spread along the walls of colon 107 behind (to the left of) the withdrawing balloon 408. This coating of wall 107 with barium is achieved by the sliding motion of balloon 408 along colon wall 107. Gas can be pumped into colon 107 behind (to the left of) the withdrawing balloon 408. Then, the barium coating of the walls of colon 107 creates a special contrast, similar to that in "air contrast barium enema". One advantage of the arrangement in Fig. 11 over conventional "air contrast barium enema" is that, in Fig. 11, the coating of the walls with barium can be controlled by adjusting the size and shape of balloon 408. For example, the smaller balloon 408, the thicker will be the barium coating on the wall behind the balloon, generally. In conventional "air contrast barium enema", barium is pushed along the colon by pumping gas, such as air, into the colon. Correspondingly, the conventional barium coating formation is less tunable than that in the technique shown in Fig. 11.

In Fig. 11, the contrast agent, such as barium, is delivered via a delivery tube 86 through opening 89 into the colon. Delivery tube 86 may be attached to tube 403 on the inside of tube 403. During the unfolding of tube 403 into the colon, delivery tube 86 and tube 403 unfold together. This is because tubes 103 and 86 are attached to each other along their length. Before delivery tube 86 and tube 403 unfold, they are packed together. An additional tube, similar to delivery tube 86, can be used for pumping gas into the part of the colon behind (to the left of) the withdrawing balloon 408.

It will be appreciated that the device and method of Fig. 11 can be used for procedures and in locations other than colon enema, such as introducing medications. Different materials can be provided through the delivery tube in or on the folded tube, which are extendable into other lumen or pipes, with or without spreading on the walls of the lumen. Similarly, once the folded tube has been unfolded to the desired location, gentle suction can be applied to the delivery tube, if it is formed of sufficiently strong material, permitting withdrawal of liquids or fluids from various locations inside the lumen.

According to another embodiment of the invention, the unfolded tube, itself, can be used to massage a wall of a lumen. In this case, the tube may be of such dimensions that it conforms to the walls of the lumen, or a portion of the lumen can be massaged at one time. Massage can be provided by moving the extended tube, itself, by sending waves through the fluid that fills the extended tube or by moving massaging objects, such as inflated balloons, by means of or through the fluid that fills the extended tube.

The foregoing description of a preferred embodiment and best mode of the invention known to applicant at the time of filing the application has been presented for the purposes of illustration and description. It is not intended to be exhaustive or to limit the invention to the precise form disclosed, and obviously many modifications and variations are possible in the light of the above teaching. The embodiment was chosen and described in order to best explain the principles of the invention and its practical application to thereby enable other skilled in the art to best utilize the invention in various embodiments and with various modifications as are suited to the particular use contemplated.

The references cited herein teach many principles that are applicable to the present invention. Therefore the full contents of these publications are incorporated by reference herein where appropriate for teachings of additional or alternative details, features and/or technical background.

It is to be understood that the invention is not limited in its application to the details set forth in the description contained herein or illustrated in the drawings. The invention is capable of other embodiments and of being practiced and carried out in various ways. Those skilled in the art will readily appreciate that various modifications and changes can be applied to the embodiments of the invention as hereinbefore described without departing from its scope, defined in and by the appended claims.

## Claims

1. Apparatus for extending a tube, the apparatus comprising:
a foldable feed tube (10, 103) having a portion (19) folded inside itself, said foldable feed tube (10, 103) inside a container (13, 104) coupled to a fluid source (16);
said fluid source (16) arranged to feed fluid into said feed tube (10, 103) through an unfolded end (18) of said feed tube (10, 103) to inflate a portion of said folded portion (19) of said feed tube (10, 103), thereby unfolding and extending said folded portion (19) adjacent said unfolded portion (18) outwards from said unfolded portion (18) to become an extension of the already unfolded portion, thus extending said unfolded portion (18) lengthwise;
**characterized in that**
it comprises at least one aperture (108) in said feed tube (10, 103) permitting outflow of fluid from said feed tube (10, 103);
and **in that** an increase in length of said feed tube (10, 103) occurs due to sequential unfolding of a folded part (117) of the feed tube (10, 103) to form an unfolded part (123) of the feed tube (10, 103).

2. The apparatus according to claim 1, wherein said fluid causes said portion of said tube to unfold and extend by eversion (turning inside-out) of a folded part of said feed tube forming an unfolded part of said feed tube.

3. The apparatus according to any of the preceding claims, further comprising an object coupled to said tube for insertion as said tube unfolds; further wherein said object is selected from the group including a solid object, at least one camera, a set of cameras for producing a three-dimensional image, an ultrasound sensor, a source of ultrasound, a radiation sensor, a source of radiation.

4. The apparatus according to claim 1, further comprising:
a drainage channel configured for insertion into a rectum, said feed tube being disposed inside and extending through said drainage channel and said rectum into said colon, whereby washing liquid is drained out of said colon through said drainage channel; and
an inflatable balloon coupled to said drainage channel, which balloon is inflated inside the rectum, said inflated balloon together with said drainage channel retaining said drainage channel in said rectum.

5. The apparatus according to claim 1, further comprising at least one selected from the group of (a) a delivery tube coupled to a wall of said feed tube for extension therewith; (b) at least one aperture in said tube to permit outflow of said fluid from said tube.

## Patentansprüche

1. Vorrichtung zum Verlängern einer Röhre, die Vorrichtung umfassend:
eine faltbare Zuführröhre (10, 103) mit einem in sich selbst gefalteten Abschnitt (19), wobei die faltbare Zuführröhre (10, 103) innerhalb eines Behältnisses (13, 104) mit einer Fluidquelle (16) verbunden ist,
wobei die Fluidquelle (16) angeordnet ist, um Fluid durch ein ungefaltetes Ende (18) der Zuführröhre (10, 103) in die Zuführröhre (10, 103) zu leiten, um einen Abschnitt des gefalteten Abschnitts (19) der Zuführröhre (10, 103) aufzublasen, um hierdurch den von dem ungefalteten Abschnitt (18) benachbarten gefalteten Abschnitt (19) nach außen von dem ungefalteten Abschnitt (18) zu entfalten und zu verlängern, um eine Verlängerung des bereits ungefalteten Abschnitts (18) zu bilden, um hierdurch den ungefalteten Abschnitt (18) in Längsrichtung zu verlängern,
**dadurch gekennzeichnet, dass**
die Vorrichtung wenigstens eine Blende (108) in der Zuführröhre (10, 103) umfasst, welche den Ausfluss von Fluid aus der Zuführröhre (10, 103) zulässt,
und dass eine Zunahme an Länge der Zuführröhre (10, 103) durch sequentielles Entfalten des gefalteten Abschnitts (117) der Zuführröhre (10, 103) stattfindet, um einen ungefalteten Abschnitt (123) der Zuführröhre (10, 103) zu bilden.

2. Vorrichtung gemäß Anspruch 1, wobei das Fluid das Entfalten und Verlängern des Abschnittes der Röhre durch Eversion (Umstülpen) eines gefalteten Abschnitts der Zuführröhre verursacht, um einen ungefalteten Abschnitt der Zuführröhre zu bilden.

3. Vorrichtung gemäß irgendeinem der vorherigen Ansprüche, ferner umfassend ein mit der Röhre zur Insertion bei der Entfaltung der Röhre verbundenes Objekt, worin das Objekt ferner ausgewählt ist aus der Gruppe, beinhaltend ein festes Objekt, wenigstens eine Kamera, ein Set an Kameras zur Herstellung eines dreidimensionalen Bildes, einen Ultraschallsensor, eine Ultraschallquelle, einen Strahlungssensor, eine Strahlungsquelle.

4. Vorrichtung gemäß Anspruch 1, ferner umfassend:
eine Kanaldrainage konfiguriert zum Einsetzen in ein Rektum, wobei das Zuführrohr im Inneren angeordnet ist und sich durch die Kanaldrainage und das Rektum in den Dickdarm erstreckt, wodurch Waschflüssigkeit aus dem Dickdarm durch die Kanaldrainage entleert wird, und
einen aufblasbaren Ballon, der mit der Kanaldrainage verbunden ist, wobei der Ballon innerhalb des Rektums aufgeblasen wird, wobei der entfaltete Ballon zusammen mit der Kanaldrainage die Kanaldrainage in dem Rektum verbleibt.

5. Vorrichtung nach Anspruch 1, ferner umfassend wenigstens eines ausgewählt aus der Gruppe aus (a) einen mit einer Wand der Zuführungsröhre verbundenem Verabreichungsschlauch zu dessen Verlängerung, (b) wenigstens eine Blende in der Röhre, um das Ausfließen des Fluids aus der Röhre zuzulassen.

## Revendications

1. Appareil pour prolonger un tube, l'appareil comprenant:
un tube d'alimentation pliable (10, 103) ayant une portion (19) pliée en elle-même, ledit tube d'alimentation pliable (10, 103) à l'intérieur d'un récipient (13, 104) couplé à une source de fluide (16);
ladite source de fluide (16) arrangée pour alimenter du fluide dans ledit tube d'alimentation (10, 103) à travers une extrémité non-pliée (18) dudit tube d'alimentation (10, 103) pour gonfler une portion de ladite portion pliée (19) dudit tube d'alimentation (10, 103), ainsi dépliant et prolongeant ladite portion pliée (19) adjacente à ladite portion non-pliée (18) vers l'extérieur de ladite portion non-pliée (18) pour devenir une prolongation de la portion déjà dépliée, ainsi prolongeant ladite portion dépliée (18) longitudinalement;
**caractérisé en ce que**
il comprend au moins une ouverture (108) dans ledit tube d'alimentation (10, 103) permettant la sortie de fluide dudit tube d'alimentation (10, 103);
et **en ce qu'**une augmentation de la longueur dudit tube d'alimentation (10, 103) a lieu à cause d'un dépliage séquentiel d'une partie pliée (117) du tube d'alimentation (10, 103) pour former un partie dépliée (123) du tube d'alimentation (10, 103).

2. L'appareil selon la revendication 1, dans lequel ledit fluide cause ladite portion dudit tube à se déplier et prolonger par éversion (tournant intérieur vers extérieur) d'une partie pliée dudit tube d'alimentation formant une partie dépliée dudit tube d'alimentation.

3. L'appareil selon l'une quelconque des revendications précédentes, en outre comprenant un objet couplé audit tube pour une insertion quand ledit tube se déplie; dans lequel en outre ledit objet est choisi parmi le groupe incluant un objet solide, au moins une caméra, un ensemble de caméras pour produire une image tridimensionnelle, un capteur à ultrasons, une source à ultrasons, un capteur de rayonnement, une source de rayonnement.

4. L'appareil selon la revendication 1, en outre comprenant:
un canal de drainage configuré pour une insertion dans un rectum, ledit tube d'alimentation étant disposé à l'intérieur et se prolongeant à travers ledit canal de drainage et ledit rectum dans ledit côlon, à cause duquel du liquide de lavage est évacué dudit colon à travers ledit canal de drainage; et
un ballon gonflable couplé audit canal de drainage, lequel ballon est gonflé à l'intérieur du rectum, ledit ballon gonflé ensemble avec ledit canal de drainage retenant ledit canal de drainage dans ledit rectum.

5. L'appareil selon la revendication 1, en outre comprenant au moins un choisi parmi le groupe de (a) un tube de délivrance couplé à une paroi dudit tube d'alimentation pour une prolongation avec celui-ci; (b) au moins une ouverture dudit tube pour permettre la sortie dudit fluide dudit tube.
